# EUROPÄISCHE PATENTANMELDUNG

(11) **EP 4 306 091 A1**
(43) Veröffentlichungstag der Anmeldung: **17.01.2024**
(21) Anmeldenummer: 23184801.1
(22) Anmeldetag: 11.07.2023
(51) Int. Cl.: A61F 13/20, A61F 13/34, A61F 13/551, B65D 81/20

(54) **SOFT-TAMPON, SOFT-TAMPON-BAUGRUPPE, VERFAHREN ZUM VERPACKEN EINES SOFT-TAMPONS SOWIE VAKUUMVERPACKTE SOFT-TAMPON-BAUGRUPPE**

(30) Priorität: 13.07.2022 DE 202022001611 U; 14.12.2022 DE 102022133336
(71) Anmelder: nevernot GmbH, 10999 Berlin (DE)
(72) Erfinder: KÖSSEL, Anna, 10999 Berlin (DE); TREBITSCH, Katharina, 10999 Berlin (DE)
(74) Vertreter: Prinz & Partner mbB

(57) **Zusammenfassung**

Ein Soft-Tampon (10) mit einem Körper (12) aus einem Schaumstoff hat einen Außenkonturabschnitt (16, 18, 20, 22) an einer Oberseite (25) des Körpers (12), der ein Einführende (26) des Körpers (12) definiert. Der Körper (12) weist eine Lasche (30) auf, die einem Rückholende (28) des Körpers (12) zugeordnet ist, wobei das Rückholende (28) entlang einer Längsachse des Körpers (12) zum Einführende (26) entgegengesetzt ist. Der erfindungsgemäße Soft-Tampon (10) ist dadurch gekennzeichnet, dass der Körper (12) an zumindest einer Seite über mehrere aneinander angrenzende abgerundete Außenkonturabschnitte (16, 18, 20, 22) verfügt, sodass ein Greifabschnitt (24) zwischen zwei angrenzenden abgerundeten Außenkonturabschnitten (16, 18, 20, 22) ausgebildet ist.

Ferner werden eine Soft-Tampon-Baugruppe, ein Verfahren zum Verpacken eines Soft-Tampons in einem vakuumierten Sachet sowie eine vakuumverpackte Soft-Tampon-Baugruppe angegeben.

## Beschreibung

Die Erfindung betrifft einen Soft-Tampon sowie ein Verfahren zum Verpacken eines Soft-Tampons. Ferner betrifft die Erfindung eine Soft-Tampon-Baugruppe sowie eine vakuumverpackte Soft-Tampon-Baugruppe.

Menstruationsprodukte, die als Soft-Tampon bezeichnet werden, wurden ursprünglich insbesondere auf die Nutzung während des Geschlechtsverkehrs ausgelegt und können durch ihr weiches Material unter anderem Schmerzen beim Einführen reduzieren. Zudem wird der Tragekomfort erhöht, was in besonderem Maße Endometriose- und anderen Schmerzpatient*innen zugutekommt. Diese leiden oft unter Beschwerden bei der Verwendung von herkömmlichen Baumwoll-Tampons. Auch sind herkömmliche Tampons nicht für die Nutzung beim Geschlechtsverkehr geeignet.

Bekannte Soft-Tampons weisen, im Gegensatz zu herkömmlichen Baumwoll-Tampons, keinen Rückholfaden auf. Daher eignen sich Soft-Tampons insbesondere beim Tragen knapper Badebekleidung und für die Verwendung in der Sauna. Da kein Rückholfaden vorhanden ist, ist jedoch das Entfernen des Soft-Tampons erschwert.

Ein weiterer Nachteil bekannter Soft-Tampons ist deren im Vergleich zu herkömmlichen Baumwoll-Tampons größeres Volumen, das ein größeres Verpackungsmaß bzw. Verpackungsvolumen zur Folge hat. Dies verschlechtert die Handhabung des Soft-Tampons und erhöht Lager- und Transportkosten. Unter anderem aus diesem Grund sind Soft-Tampons bisher im Einzelhandel nicht verbreitet.

Aufgabe der Erfindung ist es, einen Soft-Tampon bereitzustellen, der leicht zu handhaben ist. Insbesondere soll ein Soft-Tampon mit kleinem Verpackungsmaß bereitgestellt werden.

Die Aufgabe wird gelöst durch einen Soft-Tampon mit einem Körper aus einem Schaumstoff, wobei ein Außenkonturabschnitt an einer Oberseite des Körpers ein Einführende des Körpers definiert. Der Körper weist eine Lasche auf, die einem Rückholende des Körpers zugeordnet ist, wobei das Rückholende entlang einer Längsachse des Körpers zum Einführende entgegengesetzt ist. Der erfindungsgemäße Soft-Tampon ist dadurch gekennzeichnet, dass der Körper an zumindest einer Seite über mehrere aneinander angrenzende abgerundete Außenkonturabschnitte verfügt, sodass ein Greifabschnitt zwischen zwei angrenzenden abgerundeten Außenkonturabschnitten ausgebildet ist.

Der Greifabschnitt des Körpers vereinfacht die Handhabung des erfindungsgemäßen Soft-Tampons, indem ein zusätzlicher Soll-Angriffspunkt am Soft-Tampon ausgebildet ist, an dem der Soft-Tampon ergriffen und bewegt werden kann. Auf diese Weise ist der Soft-Tampon sowohl beim Einführen in als auch beim Entfernen aus der Vagina einfach und zuverlässig zu handhaben. Zudem kann auch im Fall, dass sich der Soft-Tampon während der Benutzung verdreht, der Soft-Tampon einfach ergriffen und entfernt bzw. dessen Position korrigiert werden.

Neben dem Greifabschnitt dient die dem Rückholende des Körpers zugeordnete Lasche zum Entfernen des Soft-Tampons. So kann eine Nutzerin*ein Nutzer in die Lasche eingreifen, um den Soft-Tampon mittels einer Zugbewegung entfernen zu können.

Das Einführende und das Rückholende bezeichnen die angedachte Ausrichtung des Soft-Tampons beim Einführen und Entfernen des Soft-Tampons.

Der Begriff "abgerundet" ist im Sinne der Erfindung auch als "gebogen" zu verstehen.

In einer Ausgestaltung weist der Körper eine Wolkenform auf. Eine Wolkenform zeichnet sich dadurch aus, dass zumindest der Großteil aller Außenkonturabschnitte des Körpers eine abgerundete bzw. gebogene Form aufweisen, insbesondere alle der Außenkonturabschnitte. Zudem ist über den Greifabschnitt zwischen den beiden angrenzenden abgerundeten Außenkonturabschnitten die typische Kontur einer Wolke gebildet, wodurch die Wolkenform des Körpers entsteht.

Die abgerundeten Außenkonturabschnitte sorgen für ein besonders leichtes Einführen des Soft-Tampons und reduzieren die Wahrscheinlichkeit, Mikrotraumata an der Vaginalwand beim Einführen oder Entfernen des Soft-Tampons zu verursachen.

Bevorzugt ist die Lasche eine Fadenlasche zur Aufnahme eines Rückholfadens, wobei die Fadenlasche derart ausgebildet ist, dass der Rückholfaden zerstörungsfrei lösbar an der Fadenlasche befestigbar ist.

Auf diese Weise kann eine Nutzerin*ein Nutzer je nach Bedarf den Rückholfaden an den Soft-Tampon anbringen oder nicht. So hat diese*r die Wahl, je nach Anwendungsfall, einen Soft-Tampon mit Rückholfaden (zum Beispiel bei Schmerzen oder diversen Sportarten) oder ohne Rückholfaden (zum Beispiel beim Geschlechtsverkehr, beim Tragen von Badebekleidung oder in der Sauna) zu verwenden. Somit kann ein optimaler Kompromiss zwischen einfacher Entfernbarkeit und Eignung für das jeweilige Anwendungsszenario gefunden werden.

Den Rückholfaden kann die Nutzerin*der Nutzer bei Bedarf in die Fadenlasche einfädeln und befestigen, beispielsweise verknoten, um die Entfernung des Soft-Tampons bei Bedarf erheblich zu vereinfachen.

Der Körper kann wenigstens eine Stanzstelle aufweisen, über die die Lasche gebildet ist. Anders ausgedrückt kann die Lasche dadurch im Körper des Soft-Tampons ausgebildet werden, dass der Körper (an einer Stelle) gestanzt wird. Auf diese Weise kann die Position der Lasche am Rückholende des Körpers einfach und flexibel festgelegt und die Kosten in der Herstellung des Körpers minimiert werden.

Um die Handhabung des Soft-Tampons weiter zu vereinfachen, kann der Soft-Tampon entlang der Längsachse spiegelsymmetrisch sein.

Der Soft-Tampon kann über eine ebene Vorderseite und eine parallel zur Vorderseite verlaufende Rückseite verfügen, die über die Außenkonturabschnitte miteinander verbunden sind. Auf diese Weise verfügt der Körper über kompakte Ausmaße und die Handhabung des Soft-Tampons vereinfacht sich weiter. Zudem verringert sich der Aufwand in der Herstellung des Soft-Tampons, da der Körper beispielsweise aus Schaumstoffplatten ausgestanzt werden kann.

Der Schaumstoff kann ein Polyurethan sein. Polyurethan weist ein hervorragendes Saugverhalten für Körperflüssigkeiten auf und ist gut verträglich. Zudem ist ein Polyurethanschaumstoff flexibel und weich, sodass die Gefahr Mikrotraumata in der Vaginalwand beim Einführen und Entfernen des Soft-Tampons zu verursachen weiter reduziert wird.

Die Aufgabe der Erfindung wird ferner gelöst durch eine Soft-Tampon-Baugruppe umfassend einen Rückholfaden und einen Soft-Tampon nach dem Oberbegriff des Anspruchs 1, wobei der Rückholfaden separat zum Soft-Tampon ausgebildet ist. Anders ausgedrückt ist der Rückholfaden beiliegend und kann je nach Bedarf von der Nutzerin*dem Nutzer am Körper des Soft-Tampons befestigt werden, beispielsweise in die Lasche eingefädelt.

Die Baugruppe, die beispielsweise in einer gemeinsamen Verpackung wie einem vakuumierten Sachet verpackt ist, umfasst den Rückholfaden und das Soft-Tampon, das einen Körper aus einem Schaumstoff hat, wobei ein Außenkonturabschnitt an einer Oberseite des Körpers ein Einführende des Körpers definiert, und wobei der Körper eine Lasche aufweist, die Lasche einem Rückholende des Körpers zugeordnet ist, und das Rückholende entlang einer Längsachse des Körpers zum Einführende entgegengesetzt angeordnet ist.

Der Soft-Tampon der Soft-Tampon-Baugruppe ist insbesondere ein Soft-Tampon wie zuvor beschrieben.

Zudem wird die Aufgabe der Erfindung gelöst durch ein Verfahren zum Verpacken eines Soft-Tampons mit einem Körper aus einem Schaumstoff, wobei der Soft-Tampon in einem vakuumierten Sachet verpackt wird.

Der Soft-Tampon ist insbesondere ein Soft-Tampon wie zuvor beschrieben.

Die Abmessungen des Soft-Tampons werden durch die Vakuumierung erheblich reduziert, insbesondere die Höhe des Soft-Tampons. Dies liegt insbesondere an dem komprimierbaren Material, aus dem der Körper des Soft-Tampons hergestellt ist. Bei einem klassischen Baumwolltampon wäre dies beispielsweise nicht möglich. Gleichzeitig kann sich der Soft-Tampon nachdem das Sachet geöffnet wurde wieder entfalten. Anders ausgedrückt entfaltet sich der Soft-Tampon nach dem Öffnen des Sachets langsam in seine Zielgröße, sodass das Einsetzen des Soft-Tampons aufgrund der anfangs komprimierten Größe zusätzlich erleichtert wird, wenn dieser direkt nach dem Öffnen des Sachets eingeführt wird. Insbesondere kann vorgesehen sein, dass sich der Soft-Tampon nach dem Öffnen des Sachets aus dem geöffneten Sachet heraus entfaltet, sodass der Soft-Tampon leicht entnommen werden kann.

Somit zeichnet sich der über das erfindungsgemäße Verfahren verpackte Soft-Tampon insbesondere durch sein geringes Packmaß bzw. sein geringes Verpackungsvolumen auf, das im Vergleich zu herkömmlichen Soft-Tampons, die ohne Vakuumierung verpackt wurden, deutlich reduziert ist.

Dies ermöglicht es, die Lager- und Transportkosten des Soft-Tampons erheblich zu verringern. Zudem vereinfacht sich die Handhabung des Soft-Tampons durch die Nutzerin*den Nutzer.

Das Sachet ist ein Beutel aus luftdichtem Material, sodass der durch das Vakuumieren im Inneren des Sachets hergestellte Unterdruck zumindest für einen angedachten Haltbarkeitszeitraum des Soft-Tampons beibehalten werden kann.

Das vakuumierte Sachet kann über alle geeigneten im Stand der Technik bekannten Methoden hergestellt werden.

In einer Variante umfasst das Verfahren beispielsweise folgende Schritte: Zunächst wird der Soft-Tampon mit einem Körper aus einem Schaumstoff bereitgestellt. Anschließend wird der Soft-Tampon in einem Aufnahmeraum des Sachets eingeführt, woraufhin das Sachet verschlossen und der Aufnahmeraum des Sachets vakuumiert wird.

In einer anderen Variante umfasst das Verfahren beispielsweise folgende Schritte: Zunächst wird der Soft-Tampon mit einem Körper aus einem Schaumstoff bereitgestellt und auf einer ersten Folie platziert. Anschließend wird eine zweite Folie auf dem Soft-Tampon und der ersten Folie angeordnet und auf die zweite Folie und den Soft-Tampon gepresst. Daraufhin werden die erste Folie und die zweite Folie miteinander verschweißt unter Bildung des Sachets, umfassend einen Aufnahmeraum, wobei der Soft-Tampon im Aufnahmeraum verbleibt und der Aufnahmeraum des Sachets beim Verschweißen vakuumiert wird.

In einer bevorzugten Ausgestaltung des erfindungsgemäßen Verfahrens wird zusätzlich zum Soft-Tampon ein Rückholfaden im vakuumierten Sachet verpackt. Der Rückholfaden wird in diesem Zusammenhang insbesondere getrennt vom Soft-Tampon im Sachet platziert. Somit kann die Nutzerin*der Nutzer, nach dem Öffnen des Sachets, den Rückholfaden optional am Soft-Tampon befestigen.

Ferner wird die Aufgabe gelöst durch eine vakuumverpackte Soft-Tampon-Baugruppe, erhalten nach einem Verfahren wie zuvor beschrieben.

Die vakuumverpackte Soft-Tampon-Baugruppe zeichnet sich insbesondere durch ihr geringes Verpackungsvolumen aus. Beispielsweise ist das Verpackungsvolumen der Soft-Tampon-Baugruppe in etwa so groß wie das Verpackungsvolumen eines herkömmlichen Kondoms, insbesondere bezogen auf die Höhe der vakuumverpackten Soft-Tampon-Baugruppe.

Das Sachet kann wenigstens einen Schwächungsabschnitt zum Öffnen des Sachets aufweisen, insbesondere zwei an gegenüberliegenden Außenkanten angeordnete Schwächungsabschnitte. Auf diese Weise wird das Öffnen des Sachets und somit die Handhabung der vakuumverpackten Soft-Tampon-Baugruppe erleichtert, während zugleich das Sachet aus einem widerstandsfähigen und luftdichtem Material bestehen kann, um den durch das Vakuumieren im Inneren des Sachets erzeugten Unterdruck zuverlässig aufrechterhalten zu können.

Weitere Merkmale und Eigenschaften ergeben sich aus der nachfolgenden Beschreibung beispielhafter Ausführungsformen, die nicht in einem einschränkenden Sinn verstanden werden sollen, sowie den Zeichnungen. In diesen zeigen:
- Fig. 1 einen erfindungsgemäßen Soft-Tampon,
- Fig. 2 eine Draufsicht auf eine erfindungsgemäße vakuumverpackte Soft-Tampon-Baugruppe, erhalten nach einem erfindungsgemäßen Verfahren, und
- Fig. 3 eine perspektivische Darstellung der vakuumverpackten Soft-Tampon-Baugruppe aus Fig. 2.

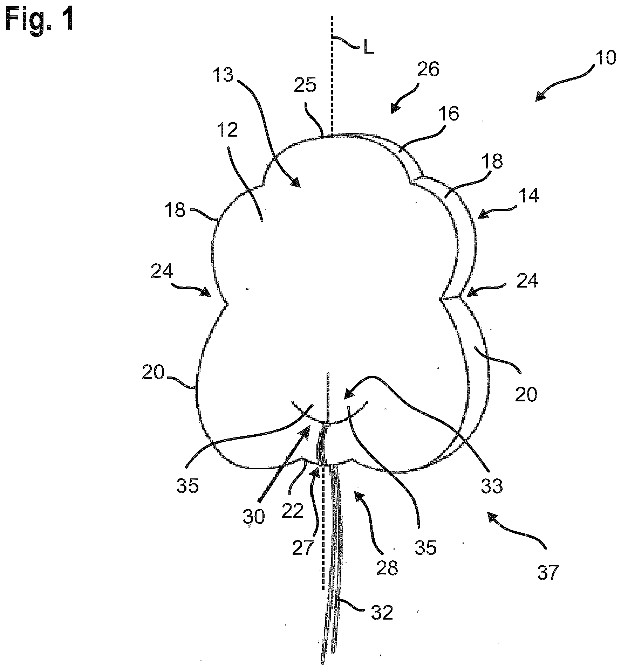

Fig. 1 zeigt einen erfindungsgemäßen Soft-Tampon 10 mit einem Körper 12 aus einem Schaumstoff.

Der Körper 12 verfügt über eine im Wesentlichen ebene Vorderseite 13 und eine parallel dazu verlaufende Unterseite 14, die über Außenkonturabschnitte 16, 18, 20 und 22 miteinander verbunden sind.

Die Außenkonturabschnitte 16 bis 22 besitzen jeweils eine abgerundete bzw. gebogene Gestalt und schließen jeweils unmittelbar aneinander an, sodass der Körper 12 insgesamt eine Wolkenform aufweist.

An den Verbindungsstellen zwischen den jeweils aneinander angrenzenden Außenkonturabschnitten 18 und 20 sind Greifabschnitte 24 ausgebildet, in die eine Nutzerin*ein Nutzer den Soft-Tampon 10 mit jeweils einen Finger ergreifen kann, um den Soft-Tampon 10 einzuführen bzw. zu entnehmen.

Durch die elastischen Eigenschaften des Schaumstoffs kann der Körper 12 auch über die Greifabschnitte 24 zusammengedrückt werden, um das Einführen des Soft-Tampons 10 zu erleichtern.

Der Außenkonturabschnitt 16 ist an einer Oberseite 25 des Körpers 12 angeordnet und definiert ein Einführende 26 des Körpers 12, während der Außenkonturabschnitt 22 an einer Unterseite 27 des Körpers 12 angeordnet ist und ein Rückholende 28 des Körpers 12 definiert.

Anders ausgedrückt wird der Körper 12 bei Benutzung des Soft-Tampons 10 insbesondere mit dem Einführende 26 voran in die Vagina der Nutzerin*des Nutzers eingeführt und mit dem Rückholende 28 aus dieser entnommen.

Das Einführende 26 und das Rückholende 28 sind entlang einer Längsachse L des Körpers 12 entgegengesetzt angeordnet. Zudem ist in Fig. 1 zu erkennen, dass der Körper 12 entlang der Längsachse L spiegelsymmetrisch ist, wobei die Spiegelebene senkrecht zur Vorderseite 13 und zur Unterseite 14 verläuft.

Der Körper 12 verfügt zudem über eine Lasche 30, die eine Fadenlasche ist, das heißt, dass ein Rückholfaden 32 über die Lasche 30 am Körper 12 befestigbar ist, wie in Fig. 1 dargestellt. Dazu wird der Rückholfaden 32 durch die Lasche 30 gefädelt und am Körper 12 verknotet. In der gezeigten Ausführungsform ist der Rückholfaden 32 zwischen der Lasche 30 und dem Außenkonturabschnitt 22 befestigt. Anders ausgedrückt ist der Rückholfaden 32 in den Körper 12 einfädelbar.

Die Lasche 30 ist als Stanzstelle 33 im Körper 12 ausgebildet, sodass der Körper 12 zwei flexible Abschnitte 35 umfasst, die wenigstens teilweise aus der durch die Vorderseite 13 und die Unterseite 14 definierten Ebenen herausbewegt werden können, um den Rückholfaden 32 in den Körper 12 einzufädeln und/oder die Lasche 30 zu ergreifen.

Dadurch, dass der Rückholfaden 32 lediglich optional am Körper 12 befestigbar ist, kann die Nutzerin*der Nutzer je nach Anwendungsfall entscheiden, ob der Rückholfaden 32 zum Einsatz kommen soll oder nicht.

Somit stellt der Soft-Tampon 10 zugleich auch eine Soft-Tampon-Baugruppe 37 dar, die den Soft-Tampon 10 sowie den separat zum Soft-Tampon 10 ausgebildeten Rückholfaden 32 umfasst.

Der erfindungsgemäße Soft-Tampon 10 zeichnet sich somit durch seine flexible Anwendbarkeit und einfache Handhabung aus.

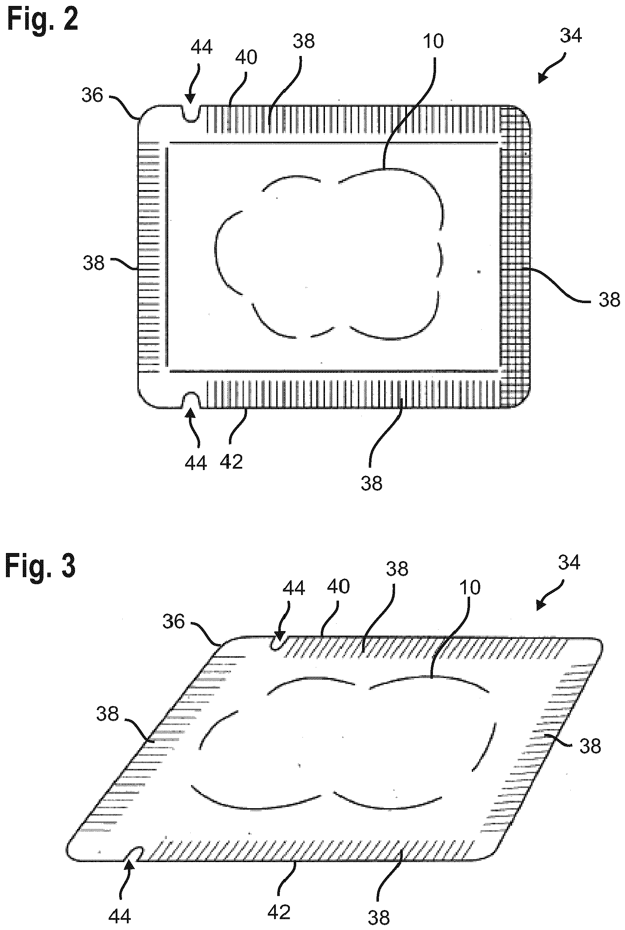

Fig. 2 stellt eine Aufsicht auf eine erfindungsgemäße vakuumverpackte Soft-Tampon-Baugruppe 34 dar.

Die vakuumverpackte Soft-Tampon-Baugruppe 34 umfasst ein Sachet 36 aus einem luftdichten Material sowie einen innerhalb des Sachets 36 aufgenommenen Soft-Tampon 10 wie zuvor beschrieben, wobei in Fig. 2 lediglich Außenkonturen des Körpers 12 des Soft-Tampons 10 angedeutet sind.

Im Inneren des Sachets 36 herrscht ein Unterdruck, der durch Vakuumieren des Sachets 36 im Herstellungsprozess der vakuumverpackten Soft-Tampon-Baugruppe 34 erzeugt worden ist.

Durch diesen Vakuumprozess ist das Verpackungsvolumen bzw. das Packmaß der vakuumierten Soft-Tampon-Baugruppe 34 erheblich reduziert, das heißt, der Soft-Tampon 10 liegt in Bezug auf die räumliche Ausdehnung des Körpers 12, insbesondere der räumlichen Ausdehnung der Außenkonturabschnitte 16 bis 22, in komprimierter Form vor.

Dies ist in Fig. 3 verdeutlicht, in der eine perspektivische Ansicht der vakuumierten Soft-Tampon-Baugruppe 34 dargestellt und zu erkennen ist, dass sich der im Sachet 36 aufgenommene Soft-Tampon 10 nicht wesentlich über die durch den Sachet 36 definierte Ebene hinaus erstreckt.

Die vakuumierte Soft-Tampon-Baugruppe 34 ist beispielsweise dadurch erhältlich, dass zunächst eine erste Folie aus dem luftdichten Material bereitgestellt wird. Auf diese wird der Soft-Tampon 10 platziert und mit einer zweiten Folie aus dem luftdichten Material abgedeckt. Die zweite Folie wird auf die erste Folie gepresst und die erste Folie und die zweite Folie unter Bildung des Sachets 36 randseitig verschweißt, sodass in Randbereichen 38 des Sachets 36 Schweißverbindungen erhalten werden, die den Sachet luftdicht verschließen. Während des Verschweißens wird der zwischen der ersten und zweiten Folie gebildete Aufnahmeraum, in dem der Soft-Tampon 10 angeordnet ist, vakuumiert, sodass der Unterdruck im Aufnahmeraum aufgebaut wird.

Um die Entnahme des Soft-Tampons 10 aus dem Sachet 36 zu erleichtern, weist das Sachet 36 an zwei gegenüberliegenden Außenkanten 40 und 42 jeweils einen Schwächungsabschnitt 44 auf. Die Nutzerin*der Nutzer kann somit das Sachet 36 öffnen, indem dieses auf Höhe der Schwächungsabschnitte 44 zerrissen wird.

Im Sachet 36, nämlich im Aufnahmeraum des Sachet 36, kann zusätzlich der Rückholfaden 32 beigelegt werden, der jedoch noch nicht am Körper 12 befestigt ist, sodass die Nutzerin*der Nutzer den Rückholfaden 32 je nach Bedarf anbringen kann oder nicht. Der Rückholfaden 32 ist somit lediglich optional beigelegt.

Die vakuumierte Soft-Tampon-Baugruppe 34 zeichnet sich insbesondere durch ihren sehr geringen Platzbedarf aus, der eine einfache Handhabung und einen geringen Lageraufwand ermöglicht.

## Patentansprüche

1. Soft-Tampon (10) mit einem Körper (12) aus einem Schaumstoff,
wobei ein Außenkonturabschnitt (16, 18, 20, 22) an einer Oberseite (25) des Körpers (12) ein Einführende (26) des Körpers (12) definiert, und
wobei der Körper (12) eine Lasche (30) aufweist, die Lasche (30) einem Rückholende (28) des Körpers (12) zugeordnet ist, und das Rückholende (28) entlang einer Längsachse des Körpers (12) zum Einführende (26) entgegengesetzt angeordnet ist, **dadurch gekennzeichnet, dass**
der Körper (12) an zumindest einer Seite über mehrere aneinander angrenzende abgerundete Außenkonturabschnitte (16, 18, 20, 22) verfügt, sodass ein Greifabschnitt (24) zwischen zwei angrenzenden abgerundeten Außenkonturabschnitten (16, 18, 20, 22) ausgebildet ist.

2. Soft-Tampon (10) nach Anspruch 1, wobei der Körper (12) eine Wolkenform aufweist.

3. Soft-Tampon (10) nach Anspruch 1 oder 2, wobei die Lasche (30) eine Fadenlasche zur Aufnahme eines Rückholfadens (32) ist, und wobei die Fadenlasche derart ausgebildet ist, dass der Rückholfaden (32) zerstörungsfrei lösbar an der Fadenlasche befestigbar ist.

4. Soft-Tampon (10) nach einem der vorhergehenden Ansprüche, wobei der Körper (12) wenigstens eine Stanzstelle (33) aufweist, über die die Lasche (30) gebildet ist.

5. Soft-Tampon (10) nach einem der vorhergehenden Ansprüche, wobei der Schaumstoff ein Polyurethan ist.

6. Soft-Tampon-Baugruppe (37) umfassend einen Rückholfaden (32) und einen Soft-Tampon (10) nach dem Oberbegriff des Anspruchs 1, wobei der Rückholfaden (32) separat zum Soft-Tampon (10) ausgebildet ist.

7. Verfahren zum Verpacken eines Soft-Tampons (10) mit einem Körper (12) aus einem Schaumstoff, wobei der Soft-Tampon (10) in einem vakuumierten Sachet (36) verpackt wird.

8. Verfahren nach Anspruch 7, wobei zusätzlich zum Soft-Tampon (10) ein Rückholfaden (32) im vakuumierten Sachet (36) verpackt wird.

9. Vakuumverpackte Soft-Tampon-Baugruppe (34), erhalten nach einem Verfahren gemäß einem der Ansprüche 7 oder 8.

10. Vakuumverpackte Soft-Tampon-Baugruppe (34), wobei das Sachet (36) wenigstens einen Schwächungsabschnitt (44) zum Öffnen des Sachets (36) aufweist, insbesondere zwei an gegenüberliegenden Außenkanten (40, 42) angeordnete Schwächungsabschnitte (44).
